# EUROPÄISCHE PATENTSCHRIFT

(11) **EP 0 706 987 B1**
(45) Veröffentlichungstag und Bekanntmachung des Hinweises auf die Patenterteilung: **03.06.1998**
(21) Anmeldenummer: 95810627.0
(22) Anmeldetag: 04.10.1995
(51) Int. Cl.: C07C 63/04, C07C 51/15

(54) **Verfahren zur Herstellung von alkylierten aromatischen Carbonsäuren und Carbonsäurehalogeniden**
Process for preparing alkylated aromatic carboxylic acids and carboxylic acid halogenides
Procédé de préparation d'acides aromatiques alkylés et de halogénures d'acides carboxyliques

(30) Priorität: 13.10.1994 CH 3078/94
(43) Veröffentlichungstag der Anmeldung: 17.04.1996
(73) Patentinhaber: Ciba Specialty Chemicals Holding Inc., 4057 Basel (CH)
(72) Erfinder: Hüsler, Rinaldo, Dr., CH-3184 Wünnewil (CH); Orban, Ivan, Dr., CH-4052 Basel (CH); Holer, Martin, CH-4312 Magden (CH)

(56) Entgegenhaltungen:
- CH-A- 619 204
- US-A- 3 138 626
- US-A- 3 520 926

## Beschreibung

Gegenstand der vorliegenden Anmeldung ist ein Verfahren zur Herstellung von alkylierten aromatischen Carbonsäuren aus alkylierten aromatischen Kohlenwasserstoffen, sowie ein Verfahren zur Herstellung der entsprechenden Carbonsäurehalogenide.

In der Technik sind bereits Methoden zur Herstellung von aromatischen sterisch gehinderten Carbonsäuren und den entsprechenden Carbonsäurehalogeniden bekannt. So wird beispielsweise in Org. Synth. Coll. Vol. 2, 583 (1943) die Umsetzung von Mesitylen mit Kohlenmonoxid in Gegenwart von AlCl₃ und CuCl beschrieben, wobei über die Aldehyd-Stufe durch Behandlung mit HNO₃ die Carbonsäure erhalten wird (Friedel-Crafts-Formylierung nach Gattermann-Koch). Eine andere Möglichkeit ist die Halocarbonylierung mit Oxalylchlorid, wie sie in Org. Synth. Coll. Vol. 5, 706 (1973) beschrieben ist. In der Gattermann-Adams-Synthese wird ein aromatischer Kohlenwasserstoff mit Zinkcyanid in Gegenwart von AlCl₃ zunächst zum Imidhydrochlorid umgesetzt, dann zum Aldehyd hydrolysiert und durch Behandlung mit HNO₃ in die entsprechende Carbonsäure überführt (Org. Synth. Coll. Vol. 3, 549 (1955)). Die Reaktion von aromatischen Kohlenwasserstoffen mit α,α-Dihalogenethern und anschliessender Hydrolyse zum Aldehyd ist aus Org. Synth. Coll. Vol. 5, 49 (1973) bekannt. In der EP-A-46 194 (1982) ist ein Verfahren beschrieben, worin ein aromatischer Kohlenwasserstoff zunächst acetyliert wird und das Reaktionsprodukt mit der Haloformreaktion in die entsprechende Carbonsäure überführt wird. In der Gattermann-Amid-Synthese wird aus einem aromatischen Kohlenwasserstoff die Carbamidverbindung durch Reaktion mit den entsprechenden Harnstoffsäurechloriden in Gegenwart von AlCl₃ hergestellt und zur Carbonsäure hydrolysiert (Annalen 244, 29, 55 (1888); Chem. Ber. 32, 1116 (1899)). In Chem. Ber. 97, 472 (1964) und Chem. Ber. 18, 873 (1885) werden aromatische Carbonsäuren durch Reaktion von aromatischen Kohlenwasserstoffen mit aromatischen Isocyanaten in Gegenwart von AlCl₃ und anschliessender Hydrolyse mit H₃PO₄ gewonnen. In der Cyanat-Synthese wird ein aromatischer Kohlenwasserstoff mit Natriumcyanat in Gegenwart von AlCl₃ zum Amid umgesetzt und mit Natriumnitrit in Essig- und Schwefelsäure in die Carbonsäure überführt (Angew. Chem. 61, 183 (1949); DE-A-584142 (1932); Houben-Weyl, Vol. 8, 381 und 432; Chem. Ber. 32, 1118 (1889)). Die Carboxylierung von Grignard-Verbindungen mit anschliessender Hydrolyse zur Carbonsäure ist in Org. Synth. Coll. Vol. 3, 551-555 (1955) beschrieben. Im US-A-3,187,057 (1965) werden aromatische Carbonsäuren mittels Friedel-Crafts-Synthese mit Tetrachlorkohlenstoff anstelle von Phosgen hergestellt. Eine weitere Möglichkeit der Synthese ist die Carbamidierung von aromatischen Kohlenwasserstoffen mit Urethan und anschliessende Hydrolyse zur Säure (Synthesis 1981, 977). Im J. Am. Chem. Soc. 80, 6393 (1958) ist die Herstellung von aromatischen Carbonsäuren durch die Hydrolyse von Estern, die über die Baeyer-Villiger-Oxidation aus den entsprechenden Ketonen erhalten werden, beschrieben. In der Cannizarro-Reaktion disproportionieren aromatische Aldehyde im basischen Medium in die entsprechenden Carbonsäuren und Alkohole (T.A. Geissmann, Org. Reactions, II, 94 (1944), J. Wiley & Sons, Inc. New York, London). Eine allgemeine Übersicht über Friedel-Crafts-Reaktionen ist in George A. Olah, Friedel-Crafts and Related Reactions, Vol. 3, Part 2, 1257-1269 (1964) zu finden. Die Carboxylierung von aromatischen Kohlenwasserstoffen mit Kohlendioxid und Aluminiumchlorid als Katalysator ist der GB-A-307 223 zu entnehmen. Die Edukte werden dabei unter Druck bei Temperaturen von 50-200°C umgesetzt. Die gleiche Reaktion führen Yuzo Fujiwara et al. mit Palladiumacetat als Katalysator durch (J. Organomet. Chem., 266, C44-C46 (1984)). Im J. Am. Chem. Soc. 62, 1428 (1940) veröffentlichten James. F. Norris und John E. Wood die Reaktion von aromatischen Kohlenwasserstoffen mit CO₂ und Aluminiumbromid als Katalysator. Die Umsetzungen erfolgten immer im Autoklaven bei erhöhten Drucken.
Obwohl, wie oben ausgeführt, eine Vielzahl von Synthesemethoden für aromatische Carbonsäuren bekannt ist, ist deren Anwendung zur Herstellung von alkylierten sterisch gehinderten aromatischen Carbonsäuren unbefriedigend. Beispielsweise tritt bei der sauren Hydrolyse von Amid- oder Ester-Zwischenstufen leicht eine Decarboxylierung der entstandenen Carbonsäure ein. Bei vielen Reaktionen, so auch Umsetzungen mit Kohlendioxid unter Druck und hohen Temperaturen, entstehen erhebliche Mengen an unerwünschten Nebenprodukten, wie z.B. die entsprechenden Benzophenonderivate. Auch im Hinblick auf den nicht unerheblichen apparativen und technischen Aufwand beim Arbeiten unter hohem Druck sind diese Reaktionen unvorteilhaft.

Alkylierte aromatische Carbonsäuren und Carbonsäurehalogenide, insbesondere -chloride, finden vielfältige Anwendungen, so auch als Zwischenprodukte zur Synthese von z.B. Klebstoffen. Solche Carbonsäuren und Carbonsäurehalogenide sind ausserdem wichtige Zwischenprodukte bei der Synthese von Photoinitiatorverbindungen, wie z.B. Monoacylphosphinoxiden (vgl. z.B. EP-A-7 508), Bisacylphosphinoxiden (vgl. z.B. EP-A-184 095), Benzophenonderivaten (vgl. z.B. EP-A-209 831) oder thermischen Katalysatoren, wie z.B. N-Acylimidazolen (vgl. z.B. EP-A-124 482). Es besteht daher ein Bedarf an einfachen, wirtschaftlichen Verfahren zur Herstellung dieser Ausgangsstoffe.

Es wurde nun gefunden, dass unter Einhaltung bestimmter Verfahrensbedingungen in der Hauptsache die erwünschten Carbonsäuren entstehen und nur geringe Anteile der unerwünschten Benzophenonderivate.
Die vorliegende Erfindung betrifft daher ein Verfahren zur Herstellung von Carbonsäuren der Formel I 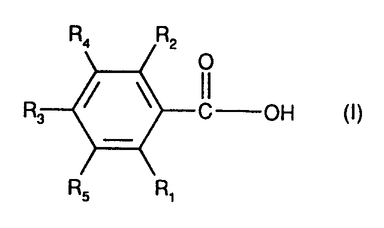
worin R₁, R₂, R₃, R₄ und R₅ unabhängig voneinander Wasserstoff, C₁-C₂₀-Alkyl, Halogen oder C₅-C₈-Cycloalkyl bedeuten, mit der Massgabe, dass mindestens zwei der Reste R₁, R₂, R₃, R₄ oder R₅ Alkyl oder/und Cycloalkyl bedeuten,
wobei aromatische Kohlenwasserstoffe der Formel (II) 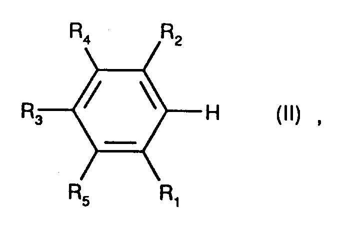
worin R₁, R₂, R₃, R₄ und R₅ die oben angegebene Bedeutung haben,
in Gegenwart eines Friedel-Krafts-Katalysators
mit Kohlendioxid umgesetzt und die entstandenen Komplexe zur entsprechenden Carbonsäure hydrolysiert werden,
dadurch gekennzeichnet, dass die Carboxylierungsreaktion bei einem Druck bis maximal 10 bar und Temperaturen von -20°C bis +40°C durchgeführt wird.

R₁, R₂, R₃, R₄ und R₅, als C₁-C₂₀-Alkyl können linear oder verzweigt sein und bedeuten beispielsweise Methyl, Ethyl, Propyl, Isopropyl, Butyl, Isobutyl, sec.-Butyl, tert.-Butyl, Pentyl, Isopentyl, Hexyl, Heptyl, Octyl, Nonyl, Decyl, Dodecyl, Octadecyl oder Icosyl. Bevorzugt sind C₁-C₁₈-, z.B. C₁-C₁₂- oder C₁-C₈-, insbesondere C₁-C₄-Alkyl. R₁, R₂, R₃, R₄ und R₅ sind insbesondere Methyl.

R₁, R₂, R₃, R₄ und R₅ als C₅-C₈-Cycloalkyl sind Cyclopentyl, Cyclohexyl, Cycloheptyl und Cyclooctyl, insbesondere Cyclopentyl und Cyclohexyl, vorzugsweise Cyclohexyl.

Halogen steht beispielsweise für Chlor, Brom und Iod, insbesondere für Chlor. Entsprechend bedeutet Halogenid Chlorid, Bromid und Iodid, insbesondere Chlorid.

Als Katalysatoren für Friedel-Crafts-Reaktionen werden üblicherweise die in George A. Olah, Friedel-Crafts and Related Reactions, Vol. I, 201 und 284-90 (1963) beschriebenen verwendet. Für das erfindungsgemässe Verfahren insbesondere geeignet sind Aluminiumtrihalogenide wie AlBr₃ und AlCl₃. Bevorzugt ist AlCl₃.

Bevorzugt ist ein Verfahren, worin die Carboxylierungsreaktion mit AlCl₃ oder AlBr₃ als Katalysator ausgeführt wird. Besonders bevorzugt ist ein Verfahren, worin die Carboxylierungsreaktion mit AlCl₃ als Katalysator ausgeführt wird.

Das erfindungsgemässe Verfahren kann ohne Lösungsmittel durchgeführt werden. So kann z.B. der aromatische Kohlenwasserstoff der Formel II, wenn er flüssig ist, selbst als Lösungsmittel dienen und wird dann im Überschuss eingesetzt. Selbstverständlich lässt sich das erfindungsgemässe Verfahren auch in inerten Lösungsmitteln durchführen. Geeignet sind beispielsweise Lösungsmittel wie sie in George A. Olah, Friedel-Crafts and Related Reactions, Vol. I, 298-302 (1963) beschrieben sind. Die Wahl des jeweiligen Lösungsmittels ist abhängig von der Löslichkeit der Edukte und Katalysatoren. Beispiele für Lösungsmittel, die im erfindungsgemässen Verfahren verwendet werden können, sind halogenierte Kohlenwasserstoffe wie Chlorbenzol, Dichlorbenzol, Tetrachlorkohlenstoff, Dichlormethan, Tetrachlorethylen, Brombenzol, aromatische Kohlenwasserstoffderivate wie z.B. Nitrobenzol, Dinitrobenzol, Benzol und Toluol, gesättigte aliphatische Kohlenwasserstoffe wie Pentan, Hexan, Heptan und deren Isomerengemische, Petrolether oder Cyclohexan oder weitere Lösungsmittel wie z.B. Kohlenstoffdisulfid, Nitroalkane, beispielsweise Nitromethan, Diethylether, Dimethylsulfoxid oder Tetramethylensulfon.
Bevorzugt sind Benzol, Toluol, Chlorbenzol und Heptan.

Eine spezielle Ausführungsform des erfindungsgemässen Verfahrens ist ein Verfahren, worin die Carboxylierungsreaktion in einem Lösungsmittel, insbesondere Toluol, Chlorbenzol oder Heptan, durchgeführt wird.

Kennzeichnend für das erfindungsgemässe Verfahren ist die Durchführung der Carboxylierungsreaktion bei niedrigen Temperaturen und ohne Überdruck bzw. mit leichtem Überdruck. Vorzugsweise wird das erfindungsgemässe Verfahren ohne Überdruck durchgeführt. Das CO₂-Gas wird mit einem Druck von maximal 10 bar, d.h. z.B. 1-10 bar in die Reaktionslösung ein- oder über dieselbe geleitet. Dabei entspricht 1 bar ca. 760 Torr, d.h. mit Reaktion bei 1 bar ist das Arbeiten bei Normaldruck, ohne Aufpressen eines Überdrucks gemeint. Insbesondere für das erfindungsgemässe Verfahren geeignet sind Drucke von 1 bis 5 bar. Im erfindungsgemässen Verfahren kann die Reaktion auch bei leichtem Unterdruck durchgeführt werden, indem z.B. die Apparatur evakuiert und dann mit CO₂-Gas entlastet wird, wobei der Gasdruck unter 1 bar gehalten wird.

Bevorzugt ist ein Verfahren, worin die Carboxylierungsreaktion ohne Überdruck durchgeführt wird.
Ebenfalls interessant ist ein Verfahren, worin die Carboxylierungsreaktion bei einem Druck von 1-5 bar durchgeführt wird.

Wie oben bereits erwähnt ist im erfindungsgemässen Verfahren ein Einleiten des CO₂-Gases in die Reaktionslösung nicht unbedingt erforderlich. Normalerweise ist es ausreichend, wenn das CO₂-Gas über der Oberfläche der Reaktionsmischung in das Reaktionsgefäss eingebracht wird. Das hat den Vorteil, dass das Einleitungsrohr nicht verstopfen kann. Übliche Operationen wie Rühren des Reaktionsgemisches sind von Vorteil.

Bevorzugt ist ein Verfahren, worin das CO₂-Gas über der Oberfläche der Reaktionsmischung, welche gerührt wird, in das Reaktionsgefäss eingebracht wird.

Wesentlich für das erfindungsgemässe Verfahren ist die Kontrolle der Temperatur, da auch bei drucklosem Einleiten von CO₂ bei zu hohen Temperaturen in der Hauptsache als unerwünschte Nebenprodukte die der jeweiligen Carbonsäure entsprechenden Benzophenon-Verbindungen entstehen.
Zweckmässig für das erfindungsgemässe Verfahren sind Temperaturen von -20°C bis +40°C, insbesondere 0°C bis 35°C, vorzugsweise Raumtemperatur, z.B. 18°C bis 25°C. Zweckmässig wird während der Durchführung der exothermen Reaktion die Reaktionsmischung mit üblichen Methoden gekühlt, um die oben beschriebenen Temperaturen einzuhalten.

Insbesondere bevorzugt ist ein Verfahren, worin die Carboxylierungsreaktion bei 0°C bis 35°C durchgeführt wird.
Interessant ist auch ein Verfahren, worin die Carboxylierungsreaktion bei Raumtemperatur durchgeführt wird.

Die Reaktionszeit ist jeweils abhängig vom verwendeten Druck. Je höher der Druck, desto kürzer sind die Reaktionszeiten. Jedoch nimmt bei zu hohen Drucken der Anteil der unerwünschten Nebenprodukte zu.

Im erfindungsgemässen Verfahren sollten die Verbindung der Formel II und der Katalysator zweckmässig mindestens in gleichen stöchiometrischen Mengen vorhanden sein. D.h. das Molverhältnis von Katalysator und aromatischem Kohlenwasserstoff sollte idealerweise 1:1 betragen. Zweckmässig ist es jedoch, eine der Komponenten im Überschuss zugegeben. So sind beispielsweise Molverhältnisse von Katalysator zu aromatischem Kohlenwasserstoff von 2:1 bis 1:10, insbesondere 2:1 bis 1:5 zweckmässig.
Wenn der aromatische Kohlenwasserstoff der Formel II auch als Lösungsmittel dient, kann der Überschuss an Kohlenwasserstoff aber auch beliebig hoch sein. Wird jedoch der Katalysator im Überschuss zugegeben, so sollte dieser zweckmässig in nicht mehr als der doppelten Menge zugegeben werden.
Wird z.B. die Carboxylierungsreaktion ohne Zugabe eines Lösungsmittels durchgeführt, so wird beispielsweise der Kohlenwasserstoff der Formel II im Überschuss zugegeben. Wenn jedoch im erfindungsgemässen Verfahren ein Lösungsmittel verwendet wird, werden zweckmässig jeweils annähernd gleiche Molmengen an Katalysator und Kohlenwasserstoff der Formel II eingesetzt.

Bevorzugt ist ein Verfahren, worin das Molverhältnis von Katalysator zu aromatischem Kohlenwasserstoff der Formel II von 2:1 bis 1:10 beträgt.

Bei der Reaktion bildet sich nicht direkt die eigentliche Carbonsäure. Vielmehr entstehen zunächst, wie dem Fachmann geläufig ist, Komplexe aus Katalysator und Säure. Aus diesen Komplexen wird durch Zusatz von Wasser die freie Säure erhalten. Günstig ist es bei der Hydrolyse zusätzlich zum Wasser Salzsäure zuzugeben.

Das CO₂ wird dem Reaktionsgemisch zweckmässig als Gas zugeführt. Dies geschieht beispielsweise durch Einleiten aus Druckbehältern oder durch Verdampfen von festem CO₂.

Insbesondere bevorzugt ist ein Verfahren, worin R₁ C₁-C₈-Alkyl, R₂ Wasserstoff oder C₁-C₄-Alkyl, R₃ Wasserstoff oder C₁-C₈-Alkyl, R₄ Wasserstoff, Chlor, Brom, Cyclohexyl oder C₁-C₈-Alkyl, und R₅ Wasserstoff oder Methyl bedeuten.

Interessant ist auch ein Verfahren worin R₁, R₂ und R₃ C₁-C₈-Alkyl bedeuten und R₄ und R₅ Wasserstoff sind.

Weiterhin bevorzugt ist ein Verfahren worin R₁, R₂ und R₃ Methyl bedeuten.

Ausserdem interessant ist ein Verfahren, worin R₁, R₂, R₃, R₄ und R₅ Methyl sind.

Die im erfindungsgemässen Verfahren als Ausgangsstoffe zu verwendenden aromatischen Kohlenwasserstoffe der Formel II sind bekannt und z.T im Handel erhältlich. Sie können auch durch dem Fachmann geläufige, in der Technik allgemein übliche Alkylierungsverfahren hergestellt werden. Solche Verfahren sind beispielsweise von Michael Novak et al. im Journal of Chemical Education, Band 70, Nr. 6, A150-154 (Juni 1993) beschrieben. Werden sie nach einem der in der Technik bekannten Verfahren hergestellt, können sie im erfindungsgemässen Verfahren auch ohne vorherige Reinigung eingesetzt werden. Das erfindungsgemässe Verfahren kann beispielsweise auch direkt ohne Isolierung der Zwischenstufe der Formel II in der Reaktionslösung aus der Herstellung dieser Vorstufe durchgeführt werden.

Die Anwendung des erfindungsgemässen Verfahrens ist beschränkt auf die Carboxylierung von mehrfach alkylierten bzw. cycloalkylierten aromatischen Kohlenwasserstoffen. Das erfindungsgemässe Verfahren ist daher nicht geeignet zur Carboxylierung von alkoxy- und hydroxy-substituierten aromatischen Kohlenwasserstoffen.

Es lassen sich mit dem erfindungsgemässen Verfahren auch am Aromaten halogenierte Carbonsäuren herstellen, indem am Aromaten halogenierte aromatische Kohlenwasserstoffe als Edukte verwendet werden. Günstiger ist es jedoch, zunächst die entsprechenden halogenfreien Carbonsäuren herzustellen und danach den Halogenrest durch Halogenierung des Aromaten einzuführen.

Die Verbindungen der Formel I können nach im Stand der Technik allgemein üblichen Verfahren in die entsprechenden Carbonsäurehalogenide überführt werden. In Org. Syntheses Coll. Vol. 3 (1955), 555-6 ist beispielsweise die Reaktion mit Thionylchlorid beschrieben. Weitere Reaktionen sind Houben-Weyl, Methoden der Organischen Chemie, Bd. VIII, Seiten 463-469 (1952), Georg Thieme Verlag, Stuttgart, sowie Houben-Weyl, Bd. E5, Seiten 593-600 (1985), Georg Thieme Verlag, Stuttgart, zu entnehmen. Die Überführung der Carbonsäuren in die entsprechenden Halogenide, insbesondere die Chloride, lässt sich auch direkt anschliessend an das erfindungsgemässe Herstellungsverfahren für die Carbonsäuren mit oder ohne deren vorherige Isolierung durchführen.

Gegenstand der Erfindung ist auch ein Verfahren zur Herstellung von Carbonsäurehalogeniden der Formel Ia 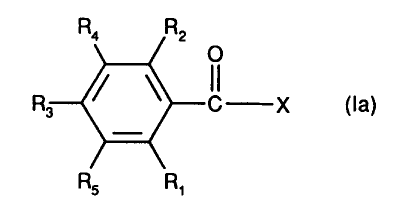
worin R₁, R₂, R₃, R₄ und R₅ unabhängig voneinander Wasserstoff, C₁-C₂₀-Alkyl, Halogen oder C₅-C₈-Cycloalkyl bedeuten und
X für Halogen steht,
mit der Massgabe, dass mindestens zwei der Reste R₁, R₂, R₃, R₄ oder R₅ Alkyl oder/und Cycloalkyl bedeuten,
wobei aromatische Kohlenwasserstoffe der Formel (II) 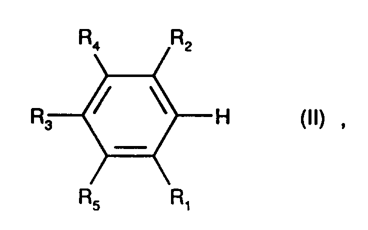
worin R₁, R₂, R₃, R₄ und R₅ die oben angegebene Bedeutung haben,
in Gegenwart eines Friedel-Krafts-Katalysators
mit Kohlendioxid umgesetzt und die entstandenen Komplexe zur entsprechenden Carbonsäure der Formel I 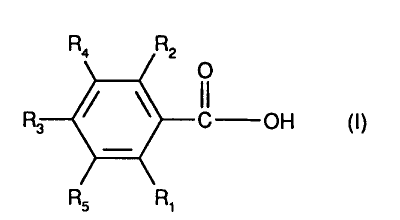 hydrolysiert werden,
und danach, gegebenenfalls nach Überführung der Säure in ein Alkalisalz, durch Reaktion mit einem Halogenierungsmittel zum Carbonsäurehalogenid der Formel Ia umgesetzt werden,
dadurch gekennzeichnet, dass die Carboxylierungsreaktion bei einem Druck bis maximal 10 bar und Temperaturen von -20°C bis +40°C durchgeführt wird und die Halogenierung mit oder ohne vorherige Isolierung der Carbonsäure der Formel I durchgeführt wird.

X als Halogen ist Cl, Br oder I, insbesondere Cl oder Br, bevorzugt Cl.

Das oben beschriebene Verfahren kann auch in einer Eintopfreaktion durchgeführt werden. Das bedeutet, dass die mit dem erfindungsgemässen Verfahren hergestellte Carbonsäure nicht in Substanz als solche isoliert und charakterisiert wird, sondern direkt in der folgenden Reaktionsstufe zum entsprechenden Halogenid, insbesondere Chlorid, umgesetzt wird. Nach der Herstellung der Carbonsäure werden in diesem Fall lediglich Wasser und die entstandenen Aluminiumsalze aus dem Reaktionsgemisch abgetrennt. Als Rückstand bleibt die entstandene Carbonsäure im überschüssigen aromatischen Kohlenwasserstoff, sofern dieser als Lösungsmittel diente, bzw. im entsprechenden Lösungsmittel gelöst. Zweckmässigerweise werden dann vor der Halogenierung noch etwaige Wasserspuren durch Andestillieren entfernt.

Natürlich kann nach dem erfindungsgemässen Verfahren die entstandene Carbonsäure zunächst auch isoliert und danach halogeniert werden.
Interessant ist ein Verfahren, worin die Halogenierung ohne Isolierung der Carbonsäurezwischenstufe erfolgt.

Geeignete Halogenierungsmittel sind beispielsweise Houben-Weyl, Methoden der Organischen Chemie, Bd. VIII, Seiten 463-469 und 475-476 (1952), Georg Thieme Verlag, Stuttgart, sowie Houben-Weyl, Bd. E5, Seiten 593-600 (1985), Georg Thieme Verlag, Stuttgart, zu entnehmen.

Geeignete Chlorierungsmittel zur Überführung von Carbonsäuren in Carbonsäurechloride sind beispielsweise anorganische Säurechloride wie z.B. Thionylchlorid, Phosphortrichlorid, Phosphorpentachlorid oder Phosgen oder Oxalylchlorid, vorzugsweise Thionylchlorid. Das Thionylchlorid wird zweckmässig im Überschuss verwendet. Zur Steigerung der Wirksamkeit von Thionylchlorid ist es zweckmässig bei der Reaktion katalytische Mengen Pyridin oder Dimethylformamid, vorzugsweise Dimethylformamid, zuzugeben.

Zur Herstellung der Bromide werden z.B. Phosphortri- oder -pentabromid eingesetzt. Die Iodide werden zweckmässig durch Umhalogenierung der entsprechenden Carbonsäurechloride erhalten.

Bevorzugt ist ein Verfahren zur Herstellung von Carbonsäurehalogeniden der Formel Ia, worin X für Cl oder Br, insbesondere Cl, steht.

Ausserdem interessant ist ein Verfahren zur Herstellung von Carbonsäurechloriden, worin X für Cl steht und als Halogenierungsmittel Thionylchlorid verwendet wird.

Es ist auch möglich die mit dem erfindungsgemässen Verfahren erhaltenen Carbonsäuren in ihre Alkalisalze zu überführen und diese mit Phosphoroxychlorid, Thionylchlorid, Phosphortrichlorid oder Phosphorpentachlorid zum entsprechenden Carbonsäurechlorid umzusetzen. Dazu wird die entstandene Carbonsäure flüssig oder fest abgetrennt. Das Salz wird dann durch Zugabe von z.B. Natriumhydroxid oder Natriumcarbonat erhalten. Es wird nach dem Fachmann geläufigen Methoden isoliert, gereinigt und getrocknet bevor es wie oben beschrieben halogeniert wird. Die Halogenierung von Natriumsalzen von Carbonsäuren ist beispielsweise in Houben-Weyl, Methoden der Organischen Chemie, Bd. VIII, Seiten 466-467 (1952), Georg Thieme Verlag, Stuttgart, beschrieben.

Nach Abdestillieren der überschüssigen Reaktanden, insbesondere des Wassers, aus der ersten Reaktionsstufe, kann vor der Halogenierung der entstandenen Carbonsäure der Formel I ein Lösungsmittel zugegeben werden. Geeignete Lösungsmittel für die Umsetzung der Carbonsäuren in die entsprechenden Carbonsäurehalogenide, insbesondere -chloride, sind nicht zu hoch siedende (gut destillierbare) Lösungsmittel, wie z.B.Toluol, Xylol, Mesitylen, Chlorbenzol usw.

Wie eingangs bereits erwähnt, sind die durch das erfindungsgemässe Verfahren herstellbaren Verbindungen wichtige Ausgangsstoffe zur Herstellung von Photoinitiatorverbindungen. Im erfindungsgemässen Verfahren können auch Isomerengemische und Gemische (vgl. Beispiel 17) von Carbonsäuren entstehen. Diese Gemische werden vorteilhafterweise nicht aufgetrennt, sondern als solche in die Halogenide, insbesondere Chloride, überführt und zu Photoinitiatoren umgesetzt. Diese Produktegemische können beispielsweise direkt bei der Herstellung von Bisacylphosphinoxid-Photoinitiatoren eingesetzt werden. Auf diese Weise werden dann Bisacylphosphinoxidgemische (beispielsweise auch Initiatoren mit zwei unterschiedlich substituierten Benzoylgruppen) erhalten. Diese Initiatorgemische weisen erniedrigte Schmelzbereiche und damit eine erhöhte Löslichkeit in den zu polymerisierenden Zusammensetzungen auf. Sie lassen sich also leichter in die Substrate einarbeiten und rekristallisieren nicht im Substrat.

Das erfindungsgemässe Verfahren bietet im Vergleich zu den meisten im Stand der Technik bekannten Verfahren, die häufig über Aldehyd- oder andere Zwischenstufen ablaufen, die Möglichkeit direkt -ohne weitere Zwischenstufen- vom aromatischen Kohlenwasserstoff zur entsprechenden Carbonsäure zu gelangen. Die durch das erfindungsgemässe Verfahren herstellbaren Carbonsäuren und Carbonsäurehalogenide sind -wie oben beschrieben- wichtige Zwischenprodukte bei der Synthese von Photoinitiatorverbindungen. Das erfindungsgemässe Verfahren bietet den Vorteil diese Zwischenstufen wirtschaftlich, ohne erheblichen apparativen und technischen Aufwand und ohne übermässige Bildung von unerwünschten Nebenprodukten herzustellen.

Die nachfolgenden Beispiele erläutern die Erfindung weiter. Angaben in Teilen oder Prozenten beziehen sich, ebenso wie in der übrigen Beschreibung und in den Patentansprüchen, auf das Gewicht, sofern nichts anderes angegeben ist.
Alle hergestellten Verbindungen werden dünnschichtchromatographisch, gaschromatographisch (GC) und ¹H-kernresonanzspektroskopisch (¹H-NMR) überprüft.

### I) Herstellung der Edukte

### Beispiel A: Herstellung von 1-Octyl-2,4,6-trimethylbenzol

In einem 750 ml Kolben werden 6,7 g (50 mmol) wasserfreies Aluminiumchlorid und 120,2 g (1 mol) Mesitylen vorgelegt. Dann werden während 2 h bei Raumtemperatur 148,7 g (1 mol) 1-Chloroctan zugetropft. Es entwickelt sich langsam HCl-Gas. Die dunkelorange Emulsion wird ca. 30 h bei Raumtemperatur nachgerührt. Die Emulsion wird auf Wasser gegossen und mit Toluol extrahiert. Die organische Phase wird abgetrennt und am Rotationsverdampfer eingeengt. Das erhaltene Öl wird im Vakuum fraktioniert destilliert. Bei einer Temperatur von 88-92°C und 1,8 mbar werden 80,6 g des Titelproduktes als farblose Flüssigkeit erhalten. Laut Gaschromatogramm (GC) und ¹H-NMR-Spektrum handelt es sich hierbei um Mono-n-octylmesitylen in einer Reinheit von 90%. Der Destillationsrückstand enthält überwiegend Dioctylmesitylen.

| Elementaranalyse: C₁₇H₂₈ | | | |
|---|---|---|---|
| ber.: | C: 91,84 % | gef.: | C: 91,67 % |
| | H: 8,16 % | | H: 8,25 % |

### Beispiel B: Herstellung von 1-Octyl-3,5-dimethylbenzol und Isomeren

Aus 106,2 g (1 mol) m-Xylol, 148,7g (1 mol) 1-Chloroctan und 6,7g (50 mmol) wasserfreiem Aluminiumchlorid werden analog zu der in Beispiel A beschriebenen Methode, jedoch ohne fraktionierte Destillation, sondern lediglich durch Abdestillieren des Lösungsmittels, nach 27 h Rühren bei Raumtemperatur 198,5 g eines Isomerengemisches des Titelproduktes als farblose Flüssigkeit erhalten. Die 4 Hauptkomponenten sind laut ¹H-NMR: 1-n-Octyl-3,5-dimethylbenzol, 1-(Methylhept-1-yl)-3,5-dimethylbenzol, 1-(Ethylhex-1-yl)-3,5-dimethylbenzol und 1-(Propylpent-1-yl)-3,5-dimethylbenzol.

| Elementaranalyse: C₁₆H₂₆ | | | |
|---|---|---|---|
| ber.: | C: 87,99 % | gef.: | C: 87,80 % |
| | H: 12,00 % | | H: 12,16 % |

### Beispiel C: Herstellung von 1-sec.-Butyl-2,4,6-trimethylbenzol

Aus 240,4 g (2 mol) Mesitylen, 185,2 g (2 mol) 2-Chlorbutan und 13,3 g (0,1 mol) wasserfreiem Aluminiumchlorid werden analog zu der in Beispiel A beschriebenen Methode nach 6 Stunden Rühren bei Raumtemperatur und Destillation bei 56°C/5 mbar 290,5 g des Titelproduktes als farblose Flüssigkeit in einer Reinheit von 90% (GC) erhalten.

| Elementaranalyse: C₁₃H₂₀ | | | |
|---|---|---|---|
| ber.: | C: 88,57 % | gef.: | C: 88,54 % |
| | H: 11,43 % | | H: 11,38 % |

### Beispiel D: Herstellung von 1-sec.-Butyl-3,5-dimethylbenzol

Aus 63,7 g (0,6 mol) m-Xylol, 46,3 g (0,5 mol) 2-Chlorbutan und 6,7 g (50 mmol) wasserfreiem Aluminiumchlorid werden analog zu der in Beispiel A beschriebenen Methode, jedoch ohne fraktionierte Destillation, sondern lediglich durch Abdestillieren des Lösungsmittels, nach 20 h Rühren bei Raumtemperatur 64,4 g des Titelproduktes als farblose Flüssigkeit in einer Reinheit von 95% (GC) erhalten.

| Elementaranalyse: C₁₂H₁₈ | | | |
|---|---|---|---|
| ber.: | C: 88,82 % | gef.: | C: 88,79 % |
| | H: 11,18 % | | H: 11,15 % |

### Beispiel E: Herstellung von 1-Cyclohexyl-2,4,5-trimethylbenzol

In einem 200 ml Kolben werden 3,3 g (0,25 mmol) wasserfreies Aluminiumchlorid und 60,1 g (0,50 mol) Mesitylen vorgelegt. Dann werden während 2,5 Stunden bei Raumtemperatur 45,2 g (0,55 mol) Cyclohexen zugetropft. Die entstehende Lösung wird noch 2 Stunden bei Raumtemperatur nachgerührt, dann auf Wasser gegossen und mit Toluol extrahiert. Die organische Phase wird abgetrennt und am Rotationsverdampfer eingeengt. Das erhaltene Öl wird im Vakuum fraktioniert destilliert.Bei einer Temperatur von 80°C und 5 mbar werden 83,1 g des Titelproduktes als farblose Flüssigkeit erhalten.Laut GC und ¹H-NMR-Spektrum handelt es sich um 1-Cyclohexyl-2,4,5-trimethylbenzol in einer Reinheit von 97,7%.

| Elementaranalyse: C₁₅H₂₂ | | | |
|---|---|---|---|
| ber.: | C: 89,04 % | gef.: | C: 88,89 % |
| | H: 10,96 % | | H: 11,09 % |

### II) Herstellung der aromatischen Carbonsäuren

### Beispiel 1: Herstellung von 2,4,6-Trimethylbenzoesäure

In einem 1,5 l Sulfierkolben werden 400 g (3,0 mol) wasserfreies Aluminiumchlorid und 432,7 g (3,6 mol) reines Mesitylen vorgelegt. Unter Rühren wird in diese Mischung bei 20°C bis 30°C drucklos CO₂-Gas eingeleitet. Während der Reaktion entsteht kein HCl-Gas. Da die Reaktion exotherm verläuft, muss gekühlt werden, um die angegebene Temperatur einzuhalten. Nach ca. 6 Stunden ist die CO₂-Aufnahme beendet. Die entstandene Suspension wird auf Eis und Salzsäurelösung gegossen, mit Hexan verdünnt, filtriert und mit Wasser und Hexan nachgewaschen. Die Kristalle werden im Vakuum bei 50°C getrocknet. Es resultieren 167,0 g weisse Kristalle (ca. 68% d. Theorie), die bei 154,0-156,1°C schmelzen. Die Reinheit der Kristalle wird dünnschichtchromatographisch und gaschromatographisch bestätigt, es sind keine Nebenprodukte mehr nachweisbar. Aus der organischen Mutterlauge werden durch Einengen weitere 10,3 g weisse Kristalle gewonnen (ca. 4% d. Theorie), diese Kristalle enthalten jedoch, gemäss gaschromatographischer Bestimmung neben der 2,4,6-Trimethylbenzoesäure 13% Hexamethylbenzophenon als Nebenprodukt. Durch Destillation lassen sich 262 g Mesitylen zurückgewinnen.

| Elementaranalyse: C₁₀H₁₂O | | | |
|---|---|---|---|
| ber.: | C: 73,15 % | gef.: | C: 73,07 % |
| | H: 7,37 % | | H: 7,36 % |

### Beispiele 2-13:

Die Verbindungen der Beispiele 2-13 werden nach dem für Beispiel 1 beschriebenen Verfahren erhalten. Die Verbindungen sowie deren Edukte und physikalische Daten sind in der folgenden Tabelle 1 zusammengefasst. Die Verbindungen werden als Rohprodukte, ohne vorherige Umkristallisation charakterisiert. Ausnahmen sind in der Tabelle gekennzeichnet. In einigen Beispielen wurde ein Lösungsmittel verwendet. Diese Beispiele und die verwendeten Lösungsmittel sind ebenfalls in der Tabelle angegeben.

**Tabelle 1**

| Bsp. | Produkt | Edukt | Schmelzpunkt [°C] | Elementaranalyse [%] ber./gef. | | |
|---|---|---|---|---|---|---|
| | | | | C | H | Halogen |
| 2 | 2,4-Dimethylbenzoesäure | 1,3-Dimethylbenzol | 123.5-127.5 | 71.98 71.98 | 6.71 6.82 | - |
| 3 | 2,3,4-Trimethylbenzoesäure | 1,2,3-Trimethylbenzol | 130-135 | 73.15 73.14 | 7.37 7.42 | - |
| 4 | 2,4,5-Trimethylbenzoesäure | 1,2,4-Trimethylbenzol | 148-150 | 73.15 72.86 | 7.37 7.37 | - |
| 5 | 2,3,4,6-Tetramethylbenzoesäure | 1,2,3,5-Tetramethylbenzol | 166-168.8 | 74.13 74.01 | 7.92 7.96 | - |
| 6 | 2,3,5,6-Tetramethylbenzoesäure * | 1,2,4,5-Tetramethylbenzol | 115-125 *¹ | 74.13 74.10 | 7.92 8.00 | - |
| 7 | 2,3,4,5,6-Pentamethylbenzoesäure * | 1,2,3,4,5-Pentamethylbenzol | 212-213.5 *² | 74.97 74.99 | 8.39 8.49 | - |
| 8 | 2-tert.-Butyl-3,6-dimethylbenzoesäure | 1-tert.-Butyl3,5-dimethylbenzol | 137-140.3 | 75.69 75.63 | 8.79 8.88 | - |
| 9 | 2,4,6-Triethylbenzoesäure | 1,3,5-Triethylbenzol | 115.8-117.2 *³ | 75.69 75.77 | 8.79 8.87 | - |
| 10 | 2,4,6-Triisopropylbenzoesäure | 1,3,5-Triisopropylbenzol | 184-188 | 77.38 77.38 | 9.74 9.77 | - |
| 11 | 2,4-Diethyl-6-methylbenzoesäure | 1,3-Diethyl-5-methylbenzol | 91-93 | 74.97 74.99 | 8.39 8.36 | - |
| 12 | 3-Chlor-2,4,6-trimethylbenzoesäure | 2-Chlor-1,3,5-trimethylbenzol | 146-146.5 | 60.46 60.43 | 5.58 5.69 | 17.85 17.81 |
| 13 | 3-Brom-2,4,6-trimethylbenzoesäure | 2-Brom-1,3,5-trimethylbenzol | 166-166.5 *² | 49.41 49.44 | 4.56 4.77 | 32.87 32.92 |

| | | | | | | |
|---|---|---|---|---|---|---|
| * Bei der Carboxylierungsreaktion wurde Toluol als Lösungsmittel verwendet | | | | | | |
| *¹ mit Natronlauge extrahiert | | | | | | |
| *² aus Ethanol umkristallisiert | | | | | | |
| *³ aus Hexan umkristallisiert | | | | | | |

### Beispiel 14: Herstellung von 1-sec.-Butyl-3,5-dimethyl-benzol und daraus 2- und 4-sec.-Butyl-2,6-dimethylbenzoesäure ohne Isolierung der Zwischenstufe

In einem 750 ml Kolben werden 13,3 g (100 mmol) wasserfreies Aluminiumchlorid und 127,4 g (1,2 mol) m-Xylol vorgelegt. Während 1,5 h werden bei 18-20°C 92,6 g (1 mol) 2-Chlorbutan zugetropft. Nach dem Zutropfen wird ca. 23 h bei 22°C nachgerührt. Im GC werden ca. 15% m-Xylol, 85% Trialkylbenzol und weniger als 1% Tetraalkylbenzol gefunden. Ohne weitere Aufarbeitung werden 133,3 g (1 mol) Aluminiumchlorid zugegeben. Während ca. 14 h wird unter Rühren bei Raumtemperatur CO₂-Gas über die Suspension geleitet. Anschliessend wird die gelborange Emulsion auf Eis und Wasser gegossen, die organische Phase mit Ether verdünnt und abgetrennt. Die Etherphase wird zweimal mit 10%iger Natronlauge extrahiert. Die Natronlaugelösung wird mit Ether versetzt und unter Kühlung mit konzentrierter Salzsäure angesäuert. Nach dem Abtrennen der wässrigen Phase wird die Etherphase mit MgSO₄ getrocknet, filtriert und am Rotationsverdampfer eingeengt. Es resultieren 46,3 g eines farblosen Öls, welches nach einigen Tagen kristallisiert. Die Kristalle weisen einen Schmelzbereich von 52-60°C auf. Laut gaschromatographischer und ¹H-NMR-Bestimmung handelt es sich bei den Kristallen um eine Mischung aus 54% 4-sec.-Butyl-2,6-dimethylbenzoesäure und 46% 2-sec.-Butyl-4,6-dimethylbenzoesäure.

| Elementaranalyse: C₁₃H₁₈O₂ | | | |
|---|---|---|---|
| ber.: | C: 75,69 % | gef.: | C: 75,67 % |
| | H: 8,79 % | | H: 8,75 % |

Aus der Etherphase, die mit Natronlauge extrahiert wurde, lassen sich Edukte und Zwischenprodukte zurückgewinnen.

### Beispiel 15: Herstellung von 2,4,6-Trimethyl-3-n-octylbenzoesäure und Isomeren

In einem 350 ml Kolben werden 54,7g (410 mmol) wasserfreies Aluminiumchlorid und 96,0 g 1-Octyl-2,4,6-trimethylbenzol (Gehalt 90%; aus Beispiel A) vorgelegt. Bei Raumtemperatur wird unter Rühren während ca. 30 h CO₂-Gas über die Reaktionsmischung geleitet. Danach wird die schwarze Emulsion auf Eis gegossen. Mit 10%iger Natronlauge wird das Benzoesäurederivat aus der organischen Phase extrahiert, mit Salzsäure angesäuert und mit Ether extrahiert. Nach dem Einengen der Etherphase und Trocknen mit MgSO₄ werden 18,7 g eines hellgelben Öles erhalten, welches zu 90% aus dem Titelprodukt besteht. Als weitere Bestandteile sind 4 % eines Isomeren und 6% Tetramethylderivat enthalten. Aus der organischen Phase lassen sich 78,2 g wiederverwendbares Edukt zurückgewinnen.

| Elementaranalyse: C₁₈H₂₈O₂ | | | |
|---|---|---|---|
| ber.: | C: 78,21 % | gef.: | C: 78,30 % |
| | H: 10,21 % | | H: 10,22 % |

### Beispiel 16: Herstellung von 3-sec.-Butyl-2,4,6-trimethylbenzoesäure

In einem 750 ml Kolben werden 213,3 g (1,6 mol) wasserfreies Aluminiumchlorid und 282,1 g 1-sec.-Butyl-2,4,6-trimethylbenzol (Gehalt 90%; aus Beispiel C) vorgelegt. Bei Raumtemperatur wird unter Rühren während ca. 48 h CO₂-Gas über die Reaktionsmischung geleitet. Danach wird die rotbraune Emulsion auf Eis gegossen. Mit 10%iger Natronlauge wird das Benzoesäurederivat aus der organischen Phase extrahiert, mit Salzsäure angesäuert und erneut mit Ether extrahiert. Das Einengen der Etherphase ergibt 44,2 g feuchte Kristalle. Nach Einbringen dieser Kristalle in Hexan, filtrieren, waschen und trocknen resultieren 11,9 g weisse Kristalle, die laut gaschromatographischer und ¹H-NMR-Bestimmung aus 60% 2,4,6-Trimethylbenzoesäure und 40% Tetramethylbenzoesäure bestehen. Aus der Hexanlösung können 31,4 g eines hellgelben Öles, welches gemäss ¹H-NMR-Spektrum und GC aus einem Gemisch aus dem Titelprodukt und Trimethylbenzoesäure (molmässig ca. je zur Hälfte) besteht, erhalten werden. Aus der organischen Phase können nichtumgesetzte Edukte zurückgewonnen werden.
Das Öl kann säulenchromatographisch aufgetrennt werden. Vorteilhafterweise wird es jedoch ohne Auftrennung zur Herstellung von Photoinitiatoren verwendet.

### Beispiel 17: Herstellung von 4,6-Dimethyl-2-octylbenzoesäure und 2,6-Dimethyl-4-octylbenzoesäure (Isomerengemisch)

In einem 750 ml Kolben werden 120 g (0,9 mol) wasserfreies Aluminiumchlorid und 195,6 g 1-Octyl-3,5-dimethylbenzol (Isomerengemisch aus Beispiel B) vorgelegt. Bei Raumtemperatur wird unter Rühren während 48 h CO₂-Gas übergeleitet. Dabei wird aus der zunächst orangen Suspension eine dunkelrote Emulsion. Das Reaktionsgemisch wird auf Eis gegossen und das Benzoesäurederivat mit 10%iger Natronlauge aus der organischen Phase extrahiert. Es entstehen drei Schichten, die getrennt werden. Die unterste Schicht ergibt nach Ansäuern mit Salzsäure und Extrahieren mit Ether 1 g Kristalle, welche laut GC ein Gemisch von Trimethylbenzoesäuren als Nebenprodukte sind. Die mittlere Schicht wird mit Ether verdünnt und mit konz. Salzsäure angesäuert. Aus der Etherphase werden beim Einengen 46,6 g gelbes Öl erhalten. Mittels einer Flashsäule (Petrolether/Essigsäureethylester 5:1) werden aus diesem Rohprodukt noch 17,7 g Edukt entfernt. Die Säurefraktion, 26,4 g gelbes Öl besteht laut GC und ¹H-NMR-Spektrum aus 62 % 4,6-Dimethyl-2-octylbenzoesäure und 38 % 2,6-Dimethyl-4-octylbenzoesäure. Die Octylgruppe ist ebenfalls isomerisiert: 7% der Produkte enthalten die 1-Octylgruppe (= n-Octyl), 64% der Produkte die 2-Octylgruppe (= 1-Methylhept-1-yl) und 29 % der Produkte die 3-Octyl (= 1-Ethylhex-1-yl) und 4-Octylgruppe (= 1-Propylpent-1-yl).

| Elementaranalyse: C₁₇H₂₆O₂ | | | |
|---|---|---|---|
| ber.: | C: 77,82 % | gef.: | C: 77,73 % |
| | H: 9,99 % | | H: 10,15 % |

### Beispiel 18: Herstellung von 3-Cyclohexyl-2,5,6-trimethylbenzoesäure

In einem 350 ml Kolben werden 44,0 g (0,33 mol) Aluminiumchlorid und 80,9 g (0,40 mol) 1-Cyclohexyl-2,4,5-trimethylbenzol (Gehalt 97,7%; aus Beispiel E) vorgelegt. Bei Raumtemperatur wird unter Rühren während ca. 17 Stunden CO₂-Gas über die Reaktionsmischung geleitet. Die dunkelbraune Lösung wird dann mit 200 ml Toluol verdünnt und auf Eis gegossen. Die beiden Phasen werden getrennt und die organische Phase wird mit 10%iger Natronlauge extrahiert. Die basische wässrige Phase wird mit Salzsäure angesäuert und mit Toluol extrahiert. Das Einengen der Toluolphase ergibt 12,3 g Kristalle. Sie werden in Petrolether und dann in Hexan-Essigsäureethylester mehrmals umkristallisiert. Die gereinigten Kristalle schmelzen bei 170-172°C. Die Struktur wird mit einem ¹H-NMR bestätigt.

| Elementaranalyse: C₁₆H₂₂O₂ | | | |
|---|---|---|---|
| ber.: | C: 78,01 % | gef.: | C: 77,92 % |
| | H: 9,00 % | | H: 8,96 % |

### Beispiel 19: Herstellung von 2,4,6-Trimethylbenzoesäure unter Verwendung von Chlorbenzol als Lösungsmittel

In einem mit Stickstoff gespülten 2,5 l Sulfierkolben werden 400 g (3,0 mol) wasserfreies Aluminiumchlorid, 432,7 g (3,6 mol) Mesitylen und 433g Chlorbenzol vorgelegt. Unter Rühren wird drucklos CO₂-Gas über die Suspension geleitet. Die Temperatur wird dabei durch schwaches Kühlen bei 20-26°C gehalten. Während der Reaktion entwickelt sich kein HCl-Gas. Nach 7 h wird die entstandene Emulsion auf Eis und Salzsäure gegossen und mit Hexan verdünnt. Die Suspension wird filtriert und die Kristalle mit Wasser und Hexan gewaschen. Nach dem Trocknen der Kristalle im Vakuum bei 70°C werden 143,9 g (58% d. Theorie) weisse Kristalle mit einem Schmelzpunkt von 153,7-155,5°C erhalten. Aus der organischen Mutterlauge werden durch fraktioniertes Einengen und Filtrieren weitere 27,1 g (11% d. Theorie) weisse Kristalle mit einem Schmelzpunkt von 153-154,7°C erhalten. Beide Kristallfraktionen enthalten laut GC kein Hexamethylbenzophenon mehr.

### Beispiel 20: Herstellung von 2,4,6-Trimethylbenzoesaure unter Verwendung von Aluminiumtribromid als Katalysator

In einem 350 ml Kolben, der mit Stickstoff gespült wurde, werden 100 g (374 mmol) wasserfreies Aluminiumtribromid (unter Heptan) vorgelegt. 54,1 g (450 mmol) Mesitylen werden zugegeben. Unter Rühren wird drucklos CO₂-Gas in den Kolben eingeleitet. Die Temperatur wird dabei durch leichtes Kühlen bei 20-30°C gehalten. Nach 2 Stunden wird die Emulsion auf Eis und Salzsäure gegossen und mit Heptan verdünnt. Die Suspension wird filtriert und mit Wasser und Heptan nachgewaschen. Nach Trocknung der Kristalle im Vakuum bei 50°C werden 17,9 g (58% d. Theorie) weisse Kristalle mit einem Schmelzpunkt von 152-154,5°C erhalten. Aus der organischen Mutterlauge werden durch fraktioniertes Einengen weitere 1,3 g (4% d. Theorie) weisse Kristalle mit einem Schmelzpunkt von 151-153°C gewonnen. Beide Kristallfraktionen enthalten gemäss GC kein Hexamethylbenzophenon mehr.

| Elementaranalyse: C₁₀H₁₂O₂ | | | |
|---|---|---|---|
| ber.: | C: 73,15 % | gef.: | C: 72,96 % |
| | H: 7,37 % | | H: 7,55 % |

### III) Herstellung der aromatischen Carbonsäurechloride

### Beispiel 21: Herstellung von 2,4,6-Trimethylbenzoesäurechlorid ohne Isolierung der Carbonsäurezwischenstufe

In einem 1 l Kolben werden 400 g (3 mol) wasserfreies Aluminiumchlorid und 432,7 g (3,6 mol) Mesitylen vorgelegt. Der Kolben wird evakuiert und dann mit CO₂-Gas entlastet. Die Suspension wird bei 18-22°C gerührt, während etwa 3 Stunden lang CO₂-Gas unter Normaldruck eingeleitet wird. Danach wird die Apparatur verschlossen und der Druck durch Zuleiten von CO₂-Gas auf 1,5 bar erhöht. Nach 5 Stunden ist die CO₂-Aufnahme beendet. Es werden insgesamt 2 mol CO₂-Gas aufgenommen. Nach dem Entlasten der Apparatur wird die entstandene Suspension auf Eis und Salzsäurelösung gegossen. Danach wird mit 350 g Mesitylen verdünnt und zum Lösen der Mesitylencarbonsäure auf ca. 80°C erwärmt. Die wässrige Phase wird bei etwa 80°C abgetrennt, und die organische Phase wird mit Wasser nachgewaschen. Letzte Wasserreste werden durch Einengen der Lösung bei Normaldruck entfernt. Es werden so 830 g organische Reaktionslösung erhalten, die ohne weitere Reinigung in der nächsten Stufe eingesetzt wird. Durch Destillation bei 88-100°C im Vakuum werden 460 g Mesitylen abdestilliert. Zum Rückstand werden 1,1 g Dimethylformamid zugegeben. Unter Rühren werden dann während 3 Stunden bei 50°C 165 g (1,4 mol) Thionylchlorid zugetropft. Nach dem Zutropfen wird etwa 1 Stunde bei 80°C bis zur Beendigung der Gasentwicklung nachreagieren lassen. Im Vakuum wird dann zunächst überschüssiges Thionylchlorid (Innentemperatur bis 115°C/7 mbar) und danach nicht umgesetztes Mesitylen (85°C Destillationstemperatur/7 mbar) abdestilliert. Anschliessend destilliert das Titelprodukt bis 100°C Destillationstemperatur/7 mbar (Anstieg der Innentemperatur auf 120°C). Es resultieren 196 g 2,4,6-Trimethylbenzoesäurechlorid mit einem Gehalt von 99% (= 71% d. Theorie) als farblose Flüssigkeit.

| Elementaranalyse: C₁₀H₁₁ClO | | | |
|---|---|---|---|
| ber:: | C: 65,76 % | gef.: | C: 65,85 % |
| | H: 6,07 % | | H: 5,49 % |
| | Cl: 19,41 % | | Cl: 19,11 % |

### IV) Herstellung eines Photoinitiators (Anwendungsbeispiel)

### Beispiel 22: Herstellung von Bis(2,4,6-trimethylbenzoyl)-isobutylohosphinoxid

Zu einer Lösung aus 31,9 ml (0,225 mol) Diisopropylamin in 80 ml Tetrahydrofuran werden unter Stickstoff bei 0°C 140,6 ml (0,225 mol; 1,6M) Butyllithium innerhalb von 30 min getropft. Diese Lösung wird bei -30°C innerhalb von 90 min zu einer Lösung aus 41,1 g (0,225 mol) 2,4,6-Trimethylbenzoylchlorid und 12 ml (0,102 mol) Isobutylphosphin in 200 ml Tetrahydrofuran getropft. Nach 2 h Rühren bei -30°C lässt man die gelbe Lösung auf Raumtemperatur erwärmen und wäscht einmal mit Wasser. Die organische Phase wird mit Magnesiumsulfat getrocknet, filtriert und am Rotationsverdampfer eingeengt. Der Rückstand wird in 200 ml Toluol gelöst und mit 11,6 g (0,102 mol) 30%igem Wasserstoffperoxid versetzt. Nach 2 h Rühren wird zuerst mit Wasser, dann mit gesättigter Natriumhydrogencarbonatlösung nachgewaschen. Danach wird mit Magnesiumsulfat getrocknet, abfiltriert und die Lösung am Rotationsverdampfer eingeengt. Nach Kristallisation aus Hexan erhält man 27,8 g (68,5% der Theorie) der oben genannten Verbindung als gelbes Pulver. Der Schmelzpunkt liegt bei 85-86°C.

| Elementaranalyse: | | | |
|---|---|---|---|
| ber.: | C: 72,34 % | gef.: | C: 72,13 % |
| | H: 7,84 % | | H: 7,94 % |

## Patentansprüche

1. Verfahren zur Herstellung von Carbonsäuren der Formel I 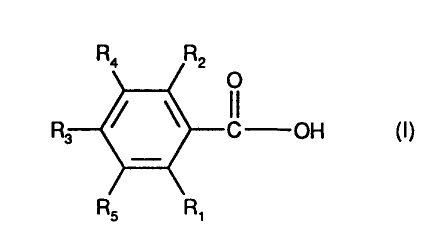
worin R₁, R₂, R₃, R₄ und R₅ unabhängig voneinander Wasserstoff, C₁-C₂₀-Alkyl, Halogen oder C₅-C₈-Cycloalkyl bedeuten, mit der Massgabe, dass mindestens zwei der Reste R₁, R₂, R₃, R₄ oder R₅ Alkyl oder/und Cycloalkyl bedeuten,
wobei aromatische Kohlenwasserstoffe der Formel (II) 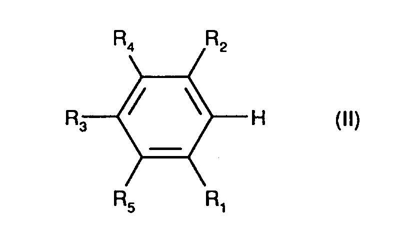
worin R₁, R₂, R₃, R₄ und R₅ die oben angegebene Bedeutung haben,
in Gegenwart eines Friedel-Krafts-Katalysators
mit Kohlendioxid umgesetzt und die entstandenen Komplexe zur entsprechenden Carbonsäure hydrolysiert werden,
dadurch gekennzeichnet, dass die Carboxylierungsreaktion bei einem Druck bis maximal 10 bar und Temperaturen von -20 bis +40°C durchgeführt wird.

2. Verfahren nach Anspruch 1, worin die Carboxylierungsreaktion ohne Überdruck durchgeführt wird.

3. Verfahren nach Anspruch 1, worin die Carboxylierungsreaktion bei einem Druck von 1 bis 5 bar durchgeführt wird.

4. Verfahren nach Anspruch 1, worin die Carboxylierungsreaktion bei 0 bis 35°C durchgeführt wird.

5. Verfahren nach Anspruch 4, worin die Carboxylierungsreaktion bei Raumtemperatur durchgeführt wird.

6. Verfahren nach Anspruch 1, worin das CO₂-Gas über der Oberfläche der Reaktionsmischung, welche gerührt wird, in das Reaktionsgefäss eingebracht wird.

7. Verfahren nach Anspruch 1, worin die Carboxylierungsreaktion in einem Lösungsmittel, insbesondere Toluol, Chlorbenzol oder Heptan, durchgeführt wird.

8. Verfahren nach Anspruch 1, worin die Carboxylierungsreaktion mit AlCl₃ oder AlBr₃ als Katalysator ausgeführt wird.

9. Verfahren nach Anspruch 8, worin die Carboxylierungsreaktion mit AlCl₃ als Katalysator ausgeführt wird.

10. Verfahren nach Anspruch 1, worin das Molverhältnis von Katalysator zu aromatischem Kohlenwasserstoff der Formel II von 2:1 bis 1:10 beträgt.

11. Verfahren nach Anspruch 1, worin R₁ C₁-C₈-Alkyl, R₂ Wasserstoff oder C₁-C₄-Alkyl, R₃ Wasserstoff oder C₁-C₈-Alkyl, R₄ Wasserstoff, Chlor, Brom, Cyclohexyl oder C₁-C₈-Alkyl, und R₅ Wasserstoff oder Methyl bedeuten.

12. Verfahren nach einem der Ansprüche 1-10, worin R₁, R₂ und R₃ C₁-C₈-Alkyl bedeuten und R₄ und R₅ Wasserstoff sind.

13. Verfahren nach Anspruch 12, worin R₁, R₂ und R₃ Methyl bedeuten.

14. Verfahren nach einem der Ansprüche 1-10, worin R₁, R₂, R₃, R₄ und R₅ Methyl sind.

15. Verfahren zur Herstellung von Carbonsäurehalogeniden der Formel Ia 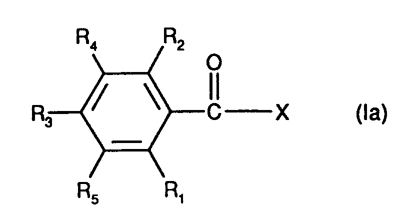
worin R₁, R₂, R₃, R₄ und R₅ unabhängig voneinander Wasserstoff, C₁-C₂₀-Alkyl, Halogen oder C₅-C₈-Cycloalkyl bedeuten und
X für Halogen steht,
mit der Massgabe, dass mindestens zwei der Reste R₁, R₂, R₃, R₄ oder R₅ Alkyl und/oder Cycloalkyl bedeuten,
wobei aromatische Kohlenwasserstoffe der Formel (II) 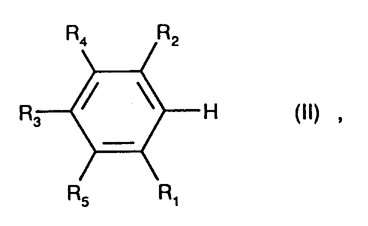
worin R₁, R₂, R₃, R₄ und R₅ die oben angegebene Bedeutung haben,
in Gegenwart eines Friedel-Krafts-Katalysators
mit Kohlendioxid umgesetzt und die entstandenen Komplexe zur entsprechenden Carbonsäure der Formel I 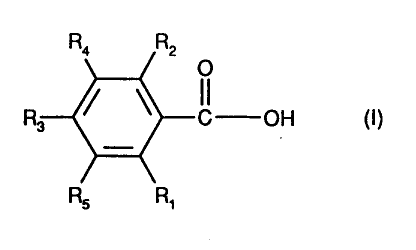 hydrolysiert werden,
und danach, gegebenenfalls nach Überführung der Säure in ein Alkalisalz, durch Reaktion mit einem Halogenierungsmittel zum Carbonsäurehalogenid der Formel Ia umgesetzt werden,
dadurch gekennzeichnet, dass die Carboxylierungsreaktion bei einem Druck bis maximal 10 bar und Temperaturen von -20 bis +40°C durchgeführt wird und die Halogenierung mit oder ohne vorherige Isolierung der Carbonsäure der Formel I durchgeführt wird.

16. Verfahren nach Anspruch 15, worin X für Cl oder Br, insbesondere Cl, steht.

17. Verfahren nach Anspruch 15, worin X für Cl steht und als Halogenierungsmittel Thionylchlorid verwendet wird.

18. Verfahren nach Anspruch 15, worin die Halogenierung ohne Isolierung der Carbonsäurezwischenstufe erfolgt.

## Claims

1. A process for the preparation of a carboxylic acid of formula I 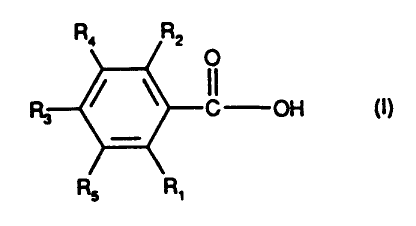
wherein R₁, R₂, R₃, R₄, and R₅ are each independently of one another hydrogen, C₁-C₂₀alkyl, halogen or C₅-C₈cycloalkyl, with the proviso that at least two of the radicals R₁, R₂, R₃, R₄ and R₅ are alkyl and/or cycloalkyl,
by reacting an aromatic hydrocarbon of formula (II) 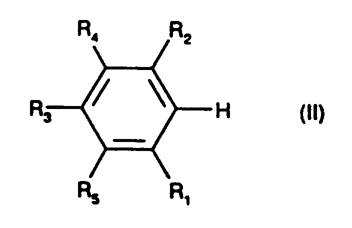
wherein R₁, R₂, R₃, R₄ and R₅ have the meanings given above, with carbon dioxide in the presence of a FriedelCrafts catalyst and hydrolysing the resulting complex to the corresponding carboxylic acid,
which process comprises carrying out the carboxylation reaction under a pressure of not more than 10 bar and at temperatures from -20 to +40°C.

2. A process according to claim 1, wherein the carboxylation reaction is carried out without applying overpressure.

3. A process according to claim 1, wherein the carboxylation reaction is carried out under a pressure of 1 to 5 bar.

4. A process according to claim 1, wherein the carboxylation reaction is carried out at O to 35°C.

5. A process according to claim 4, wherein the carboxylation reaction is carried out at room temperature.

6. A process according to claim 1, wherein the CO₂ gas is passed into the reaction vessel over the surface of the stirred reaction mixture.

7. A process according to claim 1, wherein the carboxylation reaction is carried out in a solvent, especially toluene, chlorobenzene or heptane.

8. A process according to claim 1, wherein the carboxylation reaction is carried out using AlCl₃ or AlBr₃ as catalyst.

9. A process according to claim 8, wherein the carboxylation reaction is carried out using AlCl₃ as catalyst.

10. A process according to claim 1, wherein the molar ratio of catalyst to aromatic hydrocarbon of formula II is from 2:1 to 1:10.

11. A process according to claim 1, wherein R₁ is C₁-C₈alkyl, R₂ is hydrogen or C₁-C₄alkyl, R₃ is hydrogen or C₁-C₈alkyl, R₄ is hydrogen, chloro, bromo, cyclohexyl or C₁-C₈alkyl and R₅ is hydrogen or methyl.

12. A process according to one of claims 1 to 10, wherein R₁, R₂ and R₃ are C₁-C₈alkyl, and R₄ and R₅ are hydrogen.

13. A process according to claim 12, wherein R₁, R₂ and R₃ are methyl.

14. A process according to one of claims 1 to 10, wherein R₁, R₂, R₃, R₄ and R₅ are methyl.

15. A process for the preparation of an acyl halide of formula la 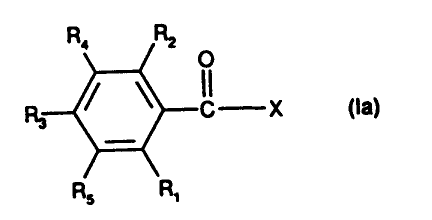
wherein R₁, R₂, R₃, R₄ and R₅ are each independently of one another hydrogen, C₁-C₂₀alkyl, halogen or C₅-C₈cycloalkyl, and
X is halogen,
with the proviso that at least two of the radicals R₁, R₂, R₃, R₄ and R₅ are alkyl and/or cycloalkyl,
by reacting an aromatic hydrocarbon of formula (II) 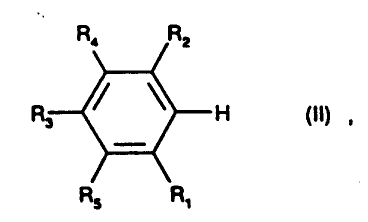
wherein R₁, R₂, R₃, R₄ and R₅ have the meanings given above, with carbon dioxide in the presence of a Friedel-Crafts catalyst and hydrolysing the resulting complex to the corresponding carboxylic acid of formula I 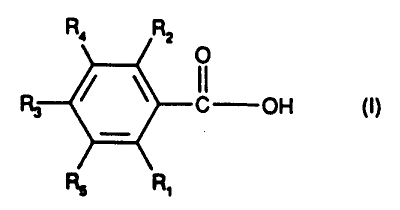
and then, after optional conversion of the acid into an alkali metal salt, reacting said acid or salt to an acyl halide of formula la by reaction with a halogenating agent,
which comprises carrying out the carboxylation reaction under a pressure of not more than 10 bar and at temperatures from -20°C to +40°C and carrying out the halogenation with or without prior isolation of the carboxylic acid of formula I.

16. A process according to claim 15, wherein X is Cl or Br, especially Cl.

17. A process according to claim 15, wherein X is Cl, and thionyl chloride is used as halogenating agent.

18. A process according to claim 15, wherein the halogenation is carried out without isolation of the carboxylic acid intermediate.

## Revendications

1. Procédé de préparation d'acide carboxylique de formule I 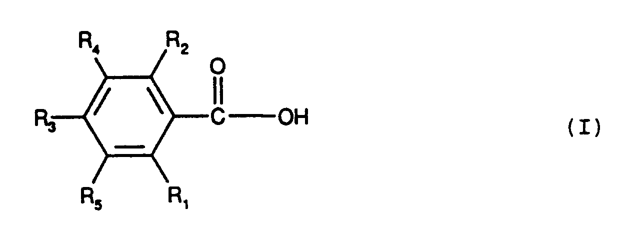
dans laquelle R₁, R₂, R₃, R₄ et R₅ représentent, indépendamment l'un de l'autre, un atome d'hydrogène, un groupe alkyle en C₁-C₂₀, un halogène ou un groupe cycloalkyle en C₅-C₈, à la condition qu'au moins deux des restes R₁, R₂, R₃, R₄ ou R₅ représentent alkyle et/ou cycloalkyle,
en faisant réagir des hydrocarbures aromatiques de formule (II) 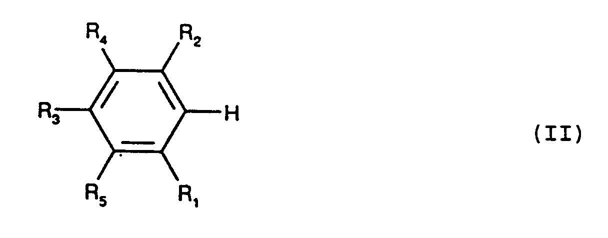
dans laquelle R₁, R₂, R₃, R₄ et R₅ ont les significations données ci-dessus,
en présence d'un catalyseur de Friedel-Crafts,
avec du dioxyde de carbone et on hydrolyse les complexes obtenus en l'acide carboxylique correspondant,
caractérisé en ce que l'on met en oeuvre la réaction de carboxylation sous une pression allant au maximum jusqu'à 10 bar et des températures de -20 à +40 °C.

2. Procédé selon la revendication 1, dans lequel on met en oeuvre la réaction de carboxylation sans surpression.

3. Procédé selon la revendication 1, dans lequel on met en oeuvre la réaction de carboxylation à une pression de 1 à 5 bars.

4. Procédé selon la revendication 1, dans lequel on met en oeuvre la réaction de carboxylation à une température de 0 à 35 °C.

5. Procédé selon la revendication 4, dans lequel on met en oeuvre la réaction de carboxylation à la température ambiante.

6. Procédé selon la revendication 1, dans lequel on introduit le CO₂ gazeux dans le réacteur à travers la surface du mélange réactionnel sous agitation.

7. Procédé selon la revendication 1, dans lequel on met en oeuvre la réaction de carboxylation dans un solvant, plus particulièrement le toluène, le chlorobenzène ou l'heptane.

8. Procédé selon la revendication 1, dans lequel on met en oeuvre la réaction de carboxylation avec AlCl₃ ou AlBr₃ comme catalyseur.

9. Procédé selon la revendication 8, dans lequel on met en oeuvre la réaction de carboxylation avec AlCl₃ comme catalyseur.

10. Procédé selon la revendication 1, dans lequel le rapport molaire du catalyseur à l'hydrocarbure aromatique de formule (II) est de 2:1 à 1:10.

11. Procédé selon la revendication 1, dans lequel R₁ représente alkyle en C₁-C₈, R₂ représente un atome d'hydrogène, ou alkyle en C₁-C₄, R₃ représente un atome d'hydrogène ou alkyle en C₁-C₈, R₄ représente un atome d'hydrogène, de chlore, de brome, un groupe cyclohexyle ou alkyle en C₁-C₈ et R₅ représente un atome d'hydrogène, ou un groupe méthyle.

12. Procédé selon l'une des revendications 1 à 10, dans lequel R₁, R₂ et R₃ représentent alkyle en C₁-C₈, et R₄ et R₅ un atome d'hydrogène.

13. Procédé selon la revendication 12, dans lequel R₁, R₂ et R₃ représentent un groupe méthyle.

14. Procédé selon l'une des revendications 1 à 10, dans lequel R₁, R₂, R₃, R₄ et R₅ représentent un groupe méthyle.

15. Procédé pour la préparation d'halogénures d'acide carboxylique de formule (Ia) 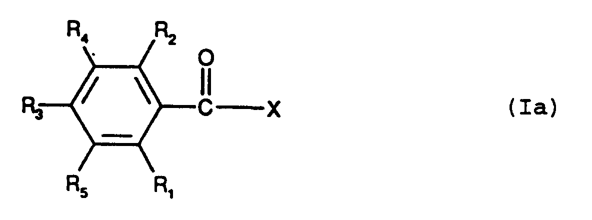
dans laquelle R₁, R₂, R₃, R₄ et R₅, indépendamment les uns des autres, représentent un atome d'hydrogène, alkyle en C₁-C₂₀, halogène ou cycloalkyle en C₅-C₈, et
X représente un atome d'halogène,
à la condition qu'au moins deux des restes R₁, R₂, R₃, R₄ ou R₅ représentent alkyle et/ou cycloalkyle,
en faisant réagir des hydrocarbures aromatiques de formule (II) 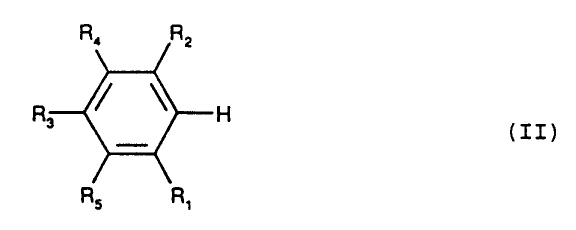
dans laquelle R₁, R₂, R₃, R₄ et R₅ ont les significations données ci-dessus,
en présence d'un catalyseur de Friedel-Crafts, avec du dioxyde de carbone, et par hydrolyse les complexes obtenus en l'acide carboxylique correspondant de formule (I) 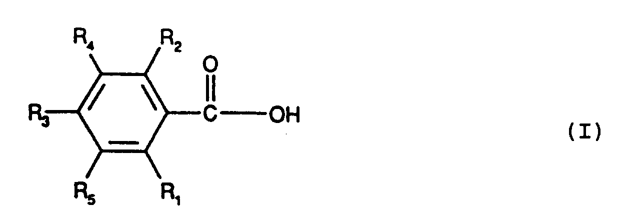
et ensuite éventuellement après transformation de l'acide en un sel alcalin, on fait réagir avec un agent d'halogénation pour obtenir l'halogénure d'acide carboxylique de formule (Ia),
caractérisé en ce que l'on met en oeuvre la réaction de carboxylation sous une pression allant jusqu'à maximum de 10 bars et dans un intervalle de températures de -20 à +40 °C, et en ce que l'on met en oeuvre l'halogénation avec ou sans isolement préalable de l'acide carboxylique de formule (I).

16. Procédé selon la revendication 15, dans lequel X représente Cl ou Br, plus particulièrement Cl.

17. Procédé selon la revendication 15, dans lequel X représente Cl et on utilise du chlorure de thionyle comme agent d'halogénation.

18. Procédé selon la revendication 15, dans lequel on met en oeuvre l'halogénation sans isolement de l'acide carboxylique intermédiaire.
